# EUROPÄISCHE PATENTSCHRIFT

(11) **EP 1 479 404 B1**
(45) Veröffentlichungstag und Bekanntmachung des Hinweises auf die Patenterteilung: **11.03.2015**
(21) Anmeldenummer: 04003927.3
(22) Anmeldetag: 20.02.2004
(51) Int. Cl.: A61M 16/00, A61M 16/10, A61M 16/16

(54) **Verfahren zur Zufuhr von Atemgas sowie Vorrichtung zur Beatmung**
Method for supplying breathable gas and breathing device
Procédé et dispositif de distribution de gaz respirable

(30) Priorität: 19.05.2003 DE 10322431
(43) Veröffentlichungstag der Anmeldung: 24.11.2004
(73) Patentinhaber: Weinmann Geräte für Medizin GmbH + Co. KG, 22525 Hamburg (DE)
(72) Erfinder: Eifler, Martin, 25348 Glückstadt (DE)
(74) Vertreter: Patentanwälte Klickow & Partner Partnerschaftsgesellschaft mbB

(56) Entgegenhaltungen:
- WO-A2-01/10489
- WO-A2-03/043560
- US-A- 4 441 027
- US-A- 4 753 758
- US-A- 4 921 642

## Beschreibung

Die Erfindung betrifft ein Verfahren zur Zufuhr von Atemgas, bei dem das Atemgas von einer Gasfördereinrichtung mit einem Förderdruck versehen wird sowie bei dem das Atemgas innerhalb eines die Gasfördereinrichtung aufnehmenden Gerätegehäuses entlang eines Luftkanals gefördert wird sowie bei dem im Bereich des Luftkanals in einem Modulschacht ein Modul zur Durchleitung des Atemgases positioniert ist, wobei das Modul für den Modulschacht mit einer in Richtung der Gasfördereinrichtung angeordneten Luftkupplung, sowie mit einer in Richtung auf einen Atemschlauch angeordneten Anschlusskupplung versehen ist und im Bereich des Moduls eine Befeuchtung des Atemgases durchgeführt wird.

Die Erfindung betrifft darüber hinaus eine Vorrichtung zur Beatmung, die eine Atemgasversorgung sowie einen Luftkanal aufweist, der innerhalb eines die Atemgasversorgung aufnehmenden Gerätegehäuses angeordnet ist, sowie bei der mindestens ein Teil des Luftkanals als ein Modulschacht ausgebildet ist, in den ein Modul zur Durchleitung des Atemgases eingesetzt ist, wobei das Modul für den Modulschacht als ein Atem-gasbefeuchter ausgebildet ist, der eine der Atemgasversorgung zugewandt angeordnete Luftkupplung sowie eine einem Atemgasschlauch zugewandt angeordnete Auslasskupplung aufweist.

Eine typische Verwendung derartiger Luftkanäle erfolgt im Zusammenhang mit Atemluftversorgungen, die im Rahmen einer CPAP-Therapie eingesetzt werden. Bei einer derartigen CPAP-Therapie (Continioüs-Positive-Airway-Pressure) wird einem Patienten im Zusammenhang mit der Therapie der Schlafapnoe wenigstens in Phasen eines Auftretens von Krankheitssymptomen Atemgas mit erhöhtem Druck zur Verfügung gestellt, um eine Abstützung des Atemweges zu erreichen.

Zur Vermeidung eines Austrocknens der Atemwege erweist es sich insbesondere bei längeren Beatmungsphasen als zweckmäßig, eine Befeuchtung der Atemluft durchzuführen. Derartige Befeuchtungen der Atemluft könnten auch bei anderen Anwendungen realisiert werden.

Typischerweise erfolgt eine Beheizung eines als Flüssigkeitsreservoir verwendeten Wassertanks des Befeuchtungssystems indirekt über einen unteren metallischen Boden des Wassertanks. Der metallische Boden wird ähnlich wie bei einem Herd auf eine Heizplatte aufgesetzt und im Bereich der Heizplatte sind elektrische Heizelemente angeordnet. Aufgrund der einzuhaltenden hygienischen Anforderungen muß der Boden zum Reinigen abnehmbar sein und trotzdem eine ausreichende Abdichtung gegen das zu erwärmende Wasser gewährleisten.

Durch die indirekte Beheizung des Wassertanks ergeben sich Energieverluste, darüber hinaus muß auch mit einer entsprechend langen Erwärmungszeit gerechnet werden. Da die Atemluftbefeuchter in der Regel im privaten Bereich der Patienten eingesetzt werden, sind zur Vermeidung entsprechend langer Heizzeiten Gegenmaßnahmen der Patienten üblich, beispielsweise das Eingießen von heißem Wasser in den Wassertank. Hieraus resultiert zum einen eine erhöhte Verletzungsgefahr des Patienten durch Hautkontakt mit dem heißen Wasser, darüber hinaus sind die Geräte nicht für eine derartige Betankung vorgesehen, so dass Beschädigungen oder Zerstörungen hervorgerufen werden können.

Ebenfalls ist es bereits bekannt, an ein Beatmungsgerät extern außenseitig einen Atemgasbefeuchter anzuschließen. Da die Atemgasbefeuchter in der Regel mit einer elektrischen Heizung ausgestattet sind, ist es bei derartigen Ausführungsformen aber erforderlich, außenseitig am Beatmungsgerät elektrische Anschlüsse vorzusehen. Eine derartige Ausführungsform wird beispielsweise in der PCT-WO 01/10489 beschrieben. Innerhalb eines Beatmungsgerätes angeordnete Atemgasbefeuchter werden beispielsweise in der PCT-WO 98/04311 beschrieben. Eine derartige Anordnung führt jedoch bei einem Betrieb des Beatmungsgerätes ohne Befeuchtung zu einem unnötig großen Gerätevolumen, wodurch der Benutzungskomfort reduziert wird.

Aus der WO 01/10489 A2 ist ein Beatmungsgerät bekannt, das mit einem Befeuchter gekoppelt ist. Der Befeuchter ist als separates Bauteil ausgeführt und neben dem Beatmungsgerät angeordnet.

Auch gemäß der US 49216642A ist ein Anfeuchter als ein separates Bauteil ausgebildet und neben einem Beatmungsgerät angeordnet.

Aufgabe der vorliegenden Erfindung ist es, ein Verfahren der einleitend genannten Art derart zu verbessern, dass sowohl eine hohe Gebrauchssicherheit als auch ein hoher Benutzungskomfort unterstützt werden.

Diese Aufgabe wird erfindungsgemäß dadurch gelöst, dass der Atemgasbefeuchter herausnehmbar in einem Modulschacht des Gerätegehäuses angeordnet ist und dass ein Heizelement innerhalb des Atemgasbefeuchters angeordnet ist.

Weitere Aufgabe der vorliegenden Erfindung ist es, ein Gerät mit kompaktem Aufbau bereitzustellen, das gleichzeitig eine hohe Gebrauchssicherheit sowie einen hohen Benutzungskomfort aufweist.

Diese Aufgabe wird erfindungsgemäß dadurch gelöst, dass der Atemgasbefeuchter herausnehmbar in einem Modulschacht des Gerätegehäuses angeordnet ist und dass ein Heizelement innerhalb des Atemgasbefeuchters angeordnet ist.

Durch die Anordnung des Moduls im Bereich des Luftkanals ist es möglich, in Abhängigkeit von den jeweils vorgegebenen Einsatzanforderungen das jeweils geeignete Modul in den Modulschacht einzusetzen. Insbesondere ist es möglich, wahlweise entweder ein Modul zur reinen Luftleitung oder ein Modul zur Luftbefeuchtung einzusetzen. Die Verwendung eines Moduls zur Luftbefeuchtung ermöglicht es insbesondere, ein Flüssigkeitsreservoir im Bereich des Luftbefeuchters mit einem räumlich getrennt zum Befeuchtungsmodul angeordneten Vorratsbehälter zu koppeln.

Die Aufteilung des Befeuchtungsvorganges und des Befeuchtungsgerätes in zwei Funktionsmodule ermöglicht die Kombination von Vorteilen, die gemäß dem Stand der Technik als nicht miteinander vereinbar angesehen wurden. Durch die voneinander räumlich getrennte Bevorratung der Befeuchtungsflüssigkeit einerseits sowie der Erhitzung der Befeuchtungsflüssigkeit andererseits ist es möglich, die bislang nicht gemeinsam realisierbaren Vorteile miteinander zu kombinieren. Die Anordnung der Heizung innerhalb des Gerätes vermeidet eine Anordnung von elektrischen Kontakten außenseitig am Gehäuse. Es sind deshalb weder aufwendige Maßnahmen erforderlich, um einen Berührungskontakt des Benutzers mit diesen elektrischen Anschlüssen zu vermeiden, noch besteht die Gefahr, dass durch Korrosionseffekte ein elektrischer Kontakt beeinträchtigt und somit die Befeuchtungsfunktion außer Betrieb gesetzt wird.

Die Heizung mit einem zugeordneten kleinen Flüssigkeitsreservoir nimmt darüber hinaus nur ein sehr kleines Volumen ein, so daß keine nennenswerte Vergrößerung des Gerätevolumens des Beatmungsgerätes mit integrierter Heizung gegenüber einem Standardgerät hervorgerufen wird. Der relativ große Vorratsbehälter mit der Befeuchtungsflüssigkeit wird nur dann an das Beatmungsgerät angekoppelt, wenn auch tatsächlich ein Befeuchtungsvorgang durchgeführt werden soll. Es wird somit stets das auf die jeweilige Anwendung abgestimmte optimale Gerätevolumen bereitgestellt.

Ein Transport der Flüssigkeit vom Vorratsbehälter zum Flüssigkeitsreservoir durch Schwerkrafteinwirkung wird dadurch unterstützt, daß der Vorratsbehälter mindestens bereichsweise in lotrechter Richtung oberhalb des Gerätegehäuses angeordnet ist.

Eine geringe resultierende Gesamthöhe des Gerätes wird dadurch unterstützt, daß der Vorratsbehälter mindestens bereichsweise seitlich neben dem Gerätegehäuse angeordnet ist.

Eine hohe Standsicherheit wird insbesondere dadurch unterstützt, daß der vorratsbehälter mindestens bereichsweise in lotrechter Richtung unterhalb des Gerätegehäuses angeordnet ist.

Eine kompakte Geräteausbildung kann auch dadurch erreicht werden, daß der Vorratsbehälter über einen Versorgungsanschluß unmittelbar an den Atemgasbefeuchter angekoppelt ist. Alternativ ist auch an die Verwendung externer Verbindungsleitungen gedacht.

Eine einfache Zusammenfügbarkeit und Trennbarkeit wird dadurch erreicht, daß der Vorratsbehälter mit einem Stutzen in einen versorgungsanschluß des Atemgasbefeuchters eingreift.

Eine erhöhte Leckagesicherheit kann dadurch bereitgestellt werden, daß der Vorratsbehälter ventilgesteuert mit dem Flüssigkeitsreservoir verbunden ist.

Die Anzahl von verwendeten Steckverbindungen kann dadurch reduziert werden, daß der Atemgasbefeuchter fest innerhalb des Grätegehäuses installiert ist.

Ein modulares Gerätekonzept wird dadurch unterstützt, daß der Atemgasbefeuchter herausnehmbar in einem Modulschacht des Gerätegehäuses angeordnet ist.

Ein wahlweiser Betrieb mit Atemgasbefeuchtung oder ohne Atemgasbefeuchtung bei gleichzeitig einfachem apparativen Aufbau kann dadurch erreicht werden, daß innerhalb des Modulschachtes ein verbindungsmodul für einen Betrieb ohne Atemgasbefeuchtung anordbar ist.

Eine einfache Ansteuerbarkeit wird dadurch unterstützt, daß die Heizung als ein elektrisches Heizelement ausgebildet ist.

Zur Verringerung von Wärmeverlusten wird vorgeschlagen, daß die Heizung als ein Heizstab ausgebildet ist.

Eine weitere Variante zur Bereitstellung einer modularen Bauweise besteht darin, daß die Heizung plattenartig ausgebildet und außerhalb des Flüssigkeitsreservoirs angeordnet ist.

Ebenfalls trägt es zu einem modularen Gerätekonzept bei, daß das Heizelement als ein vom Atemgasbefeuchter trennbares separates Bauelement ausgebildet ist.

In den Zeichnungen sind Ausführungsbeispiele schematisch dargestellt. Es zeigen:
- Fig. 1: Eine perspektivische Darstellung eines Beatmungsgerätes ohne angekoppelten Vorratsbehälter für Befeuchtungsflüssigkeit,
- Fig. 2: eine Vorderansicht eines Beatmungsgerätes mit einem Einbauraum für eine Heizung zur Luftbefeuchtung, wobei in den Einbauraum ein Kopplungselement für einen Betrieb ohne Luftbefeuchtung eingesetzt ist,
- Fig. 3: eine perspektivische Darstellung des Gerätes gemäß Fig. 2,
- Fig. 4: die Darstellung gemäß Fig. 3 mit herausgenommenem Modul,
- Fig. 5: das Gerät gemäß Fig. 2 nach einem Herausnehmen des Kopplungselementes,
- Fig. 6: einen Vertikalschnitt gemäß Schnittlinie VI-VI in Fig. 5,
- Fig. 7: das Luftleitmodul gemäß Fig. 4 in einer weiteren perspektivischen Darstellung,
- Fig. 8: eine Darstellung gemäß Blickrichtung VIII in Fig. 7,
- Fig. 9: einen Vertikalschnitt gemäß Schnittlinie IX-IX in Fig. 8,
- Fig. 10: eine perspektivische Darstellung eines Befeuchtungsmoduls zum Einsetzen in den Modulschacht gemäß Fig. 5,
- Fig. 11: eine Darstellung gemäß Blickrichtung XI in Fig. 10,
- Fig. 12: einen Vertikalschnitt gemäß Schnittlinie XII-XII in Fig. 11,
- Fig. 13: eine Vorderansicht eines gegenüber einer Darstellung in Fig. 11 abgewandelt konstruierten Befeuchters,
- Fig. 14: einen Vertikalschnitt gemäß Schnittlinie XIV-XIV in Fig. 13,
- Fig. 15: ein Horizontalschnitt gemäß Schnittlinie XV-XV in Fig. 13,
- Fig. 16: eine perspektivische Darstellung eines Beatmungsgerätes mit aufgesetztem Vorratsbehälter für die Befeuchtungsflüssigkeit,
- Fig. 17: eine vorderansicht eines Beatmungsgerätes mit einem Befeuchter, der nahe zu einer oberen Begrenzungsfläche des Beatmungsgerätes angeordnet ist,
- Fig. 18: eine perspektivische Darstellung des Gerätes gemäß Fig. 17,
- Fig. 19: eine Darstellung entsprechend Fig. 17, bei der das Luftleitungsmodul gegen einen Befeuchter ausgetauscht wurde,
- Fig. 20: eine perspektivische Darstellung des Gerätes gemäß Fig. 19,
- Fig. 21: eine Darstellung entsprechend Fig. 19 nach einem Herausnehmen des Befeuchters,
- Fig. 22: einen vertikalschnitt gemäß Schnittlinie XXII-XXII in Fig. 21,
- Fig. 23: das Gerät gemäß Fig. 19 mit aufgesetztem Vorratsbehälter,
- Fig. 24: eine perspektivische Darstellung des Gerätes gemäß Fig. 23,
- Fig. 25: einen Vertikalschnitt gemäß Schnittlinie XXV-XXV in Fig. 23,
- Fig. 26: einen vertikalschnitt gemäß Schnittlinie XXVI-XXVI in Fig. 23,
- Fig. 27: eine Vorderansicht eines Beatmungsgerätes mit einem Befeuchter, bei dem ein Heizelement durch eine wärmeübertragungsfläche mit einer verdampfungskammer verbunden ist,
- Fig. 28: einen Vertikalschnitt gemäß Schnittlinie XXVIII-XXVIII in Fig. 27,
- Fig. 29: eine Geräteansicht entsprechend Fig. 27 mit herausgenommenem Heizelement und
- Fig. 30: einen Querschnitt gemäß Schnittlinie XXX-XXX in Fig. 29.

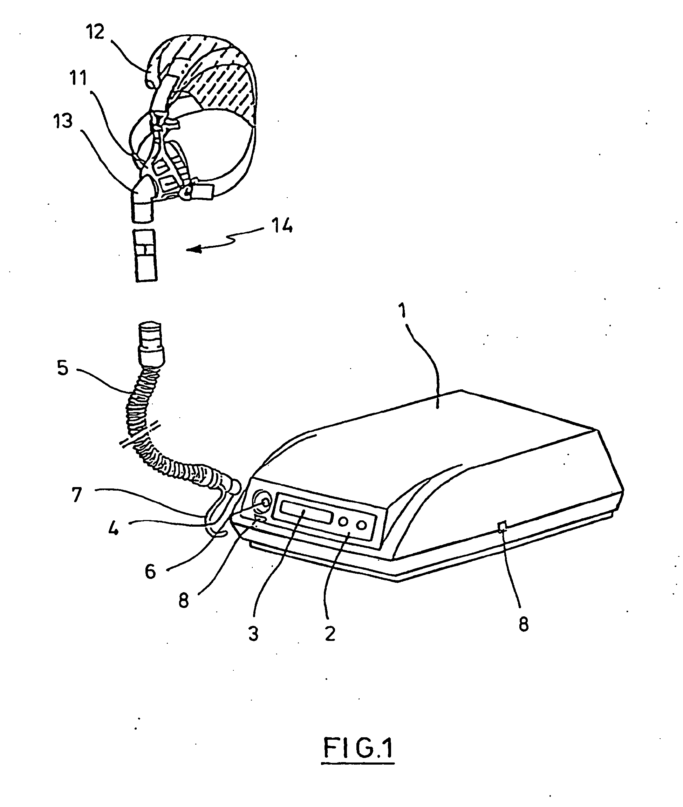

Fig. 1 zeigt den grundsätzlichen Aufbau einer Vorrichtung zur Beatmung. Im Bereich eines Beatmungsgerätes (1) mit Bedienfeld (2) sowie Anzeige (3) ist in einem Geräteinnenraum eine Atemgaspumpe angeordnet. Über eine Kopplung (4) wird ein verbindungsschlauch (5) angeschlossen. Die Kopplung (4) greift in einen Anschlußstutzen (6) des Beatmungsgerätes (1) ein. Entlang des Verbindungsschlauches (5) kann ein zusätzlicher Druckmeßschlauch (7) verlaufen, der über einen Druckeingangsstutzen (8) mit dem Beatmungsgerät (1) verbindbar ist. Zur Ermöglichung einer Datenübertragung weist das Beatmungsgerät (1) eine Schnittstelle (9) auf.

Im Bereich einer dem Beatmungsgerät (1) abgewandten Ausdehnung des Verbindungsschlauches (5) ist ein Ausatmungselement (14) angeordnet.

Fig. 1 zeigt darüber hinaus eine Beatmungsmaske (11), die als Nasalmaske ausgebildet ist. Eine Fixierung im Bereich eines Kopfes eines Patienten kann über eine Kopfhaube (12) erfolgen. Im Bereich ihrer dem verbindungsschlauch (5) zugewandten Ausdehnung weist die Beatmungsmaske (11) ein Kupplungselement (13) auf.

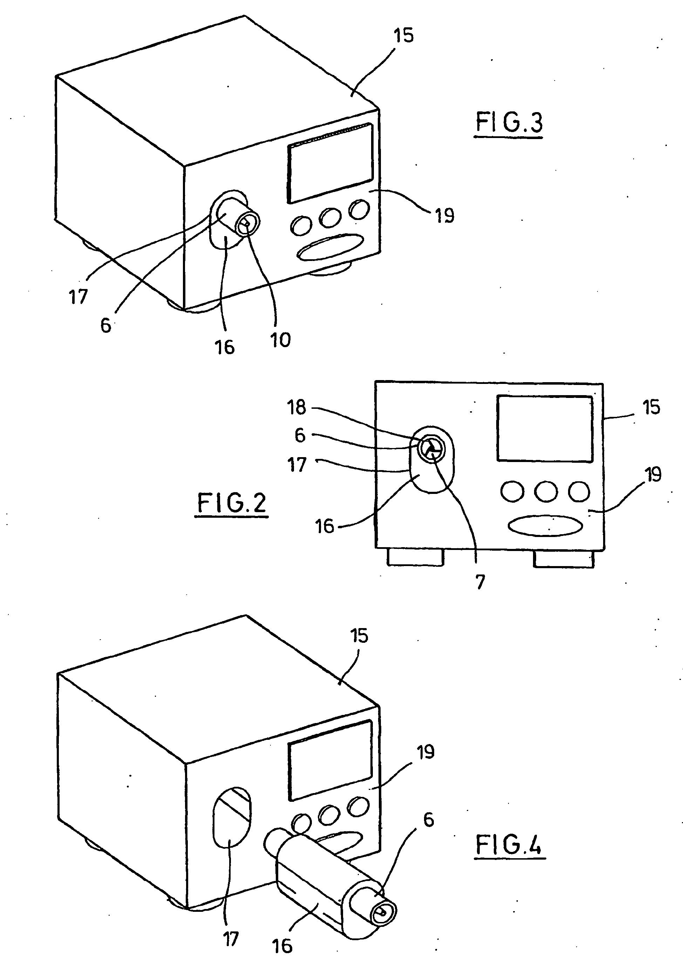

Fig. 2 zeigt ein Gerätegehäuse (15) des Beatmungsgerätes (1) in einer Vorderansicht. Es ist zu erkennen, daß der Anschlußstutzen (6) im Bereich eines verbindungsmoduls (16) angeordnet ist, das in einen Modulschacht (17) des Gerätegehäuses (15) einschiebbar ist. Im Bereich des Anschlußstutzens (6) sind eine Druckmeßleitung (10) zum Anschluß an eine innerhalb des verbindungsschlauches (5) verlaufende Druckmeßleitung (7) sowie Stützstreben (18) zur Fixierung der Druckmeßleitung (10) erkennbar.

Fig. 3 veranschaulicht die gerätetechnische Ausbildung gemäß Fig. 2 in einer perspektivischen Darstellung. Insbesondere ist zu erkennen, daß sich der Anschlußstutzen (6) über eine Frontfläche (19) des Gerätegehäuses (15) erhebt.

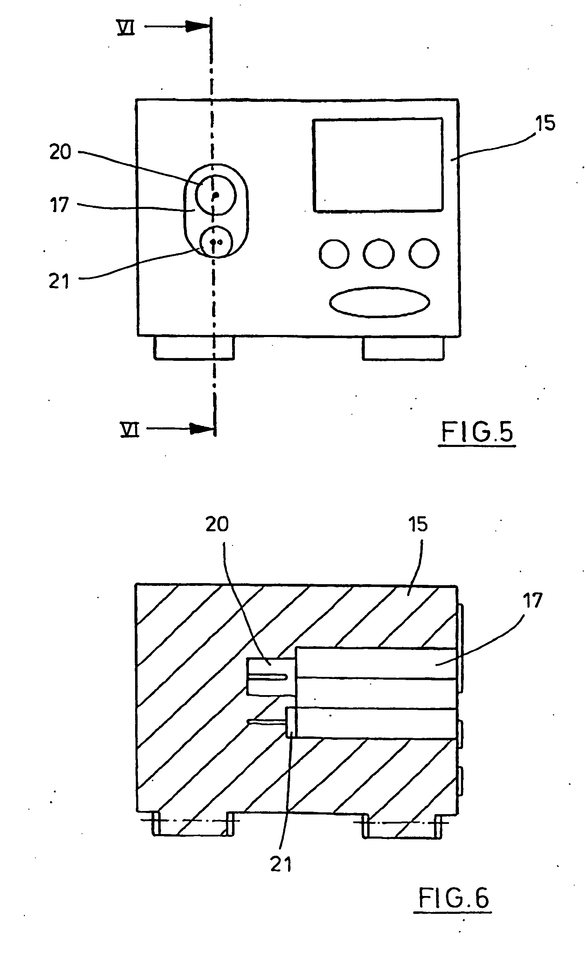

Fig. 4 zeigt das Gerät gemäß Fig. 3 nach einem Herausziehen des Verbindungsmoduls (16) aus dem Modulschacht (17). Fig. 5 zeigt eine Vorderansicht des Gerätegehäuses (15) gemäß Fig. 4 nach einer vollständigen Entfernung des Verbindungsmoduls (16). Innerhalb des Modulschachtes (17) sind eine Luftkupplung (20) sowie eine Elektrokupplung (21) zu erkennen.

Die räumliche Dimensionierung des Modulschachtes (17) sowie die Anordnung der Luftkupplung (20) sowie der Elektrokupplung (21) sind im Vertikalschnitt gemäß Fig. 6 nochmals veranschaulicht.

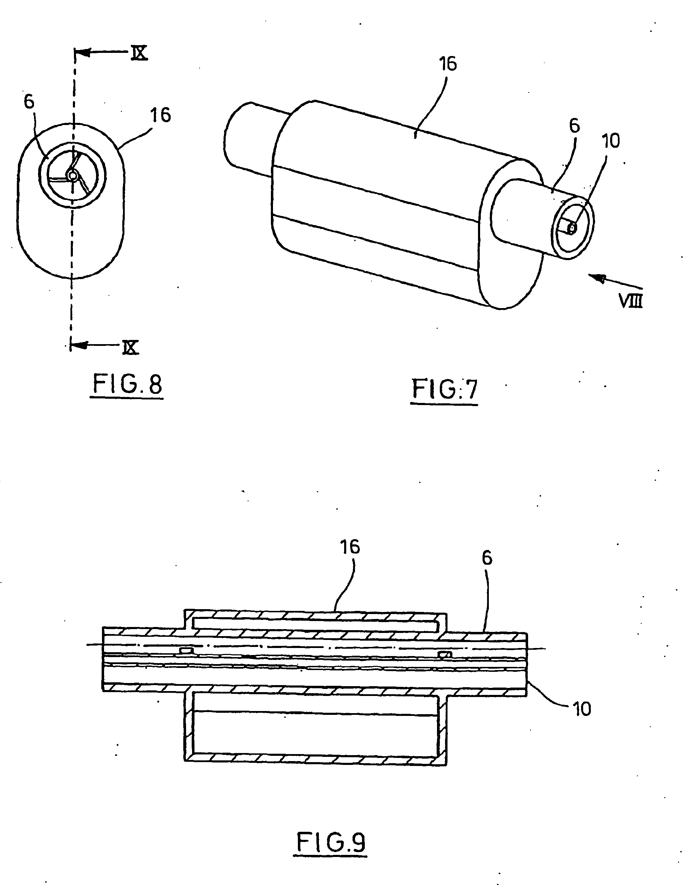

Fig. 7 und Fig. 8 zeigen das verbindungsmodul (16) zum einen in einer perspektivischen Darstellung und zum anderen in einer Vorderansicht. Fig. 9 veranschaulicht den Aufbau des Verbindungsmoduls (16) in einer Vertikalschnittdarstellung. Es ist zu erkennen, daß sich eine verlängerung des Anschlußstutzens (6) im wesentlichen tubusförmig durch das Verbindungsmodul (16) hindurch erstreckt. Ebenfalls erstreckt sich die Druckmeßleitung (10) im wesentlichen tubusförmig durch das verbindungsmodul (16) hindurch. Eine Positionierung der Druckmeßleitung. (10) erfolgt durch die Stützstreben (18).

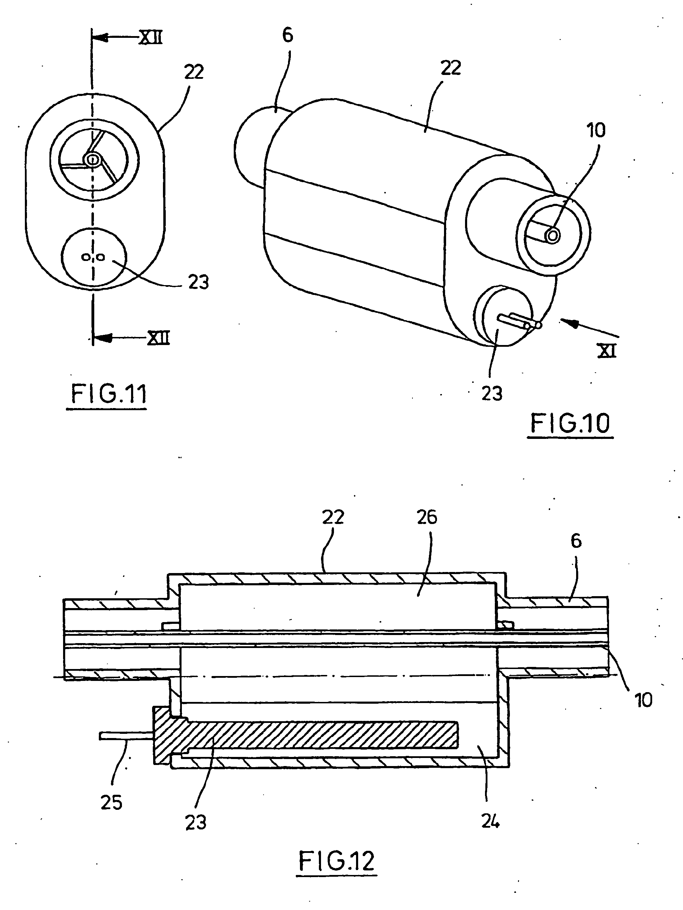

Fig. 10 zeigt einen Atemgasbefeuchter (22), der im Hinblick auf seine Außenkontur im wesentlichen an das Verbindungsmodul (16) angepaßt ist, so daß wahlweise der Atemgasbefeuchter (22) oder das Verbindungsmodul (16) im Modulschacht (17) angeordnet werden können. Der konstruktive Aufbau des Atemgasbefeuchters (22) ist in Fig. 12 durch die Vertikalschnittdarstellung weiter veranschaulicht. Es ist zu erkennen, daß ein Heizelement (23) mit einer Gestaltung ähnlich zu einem Heizstab innerhalb eines Flüssigkeitsreservoirs (24) angeordnet ist. Das Heizelement (23) ist mit einem Anschlußkontakt (25) nach einem Einschieben des Atemgasbefeuchters (22) in den Modulschacht (17) mit der Elektrokupplung (21) verbindbar. Vom Heizelement (23) verdunstete Feuchtigkeit steigt in einen Durchgangsraum (26) des Atemgasbefeuchters (22) auf und wird von dem ebenfalls durch den Durchgangsraum (26) hindurch strömenden Atemgas mitgenommen.

Der Anschlußstutzen (6) sowie die Druckmeßleitung (10) sind beim Atemgasbefeuchter (22) im wesentlichen identisch zu den entsprechenden Bauelementen beim Verbindungsmodul '(16) konstruiert. Auch hierdurch wird die wahlweise Anordnung innerhalb des Modulschachtes (17) unterstützt.

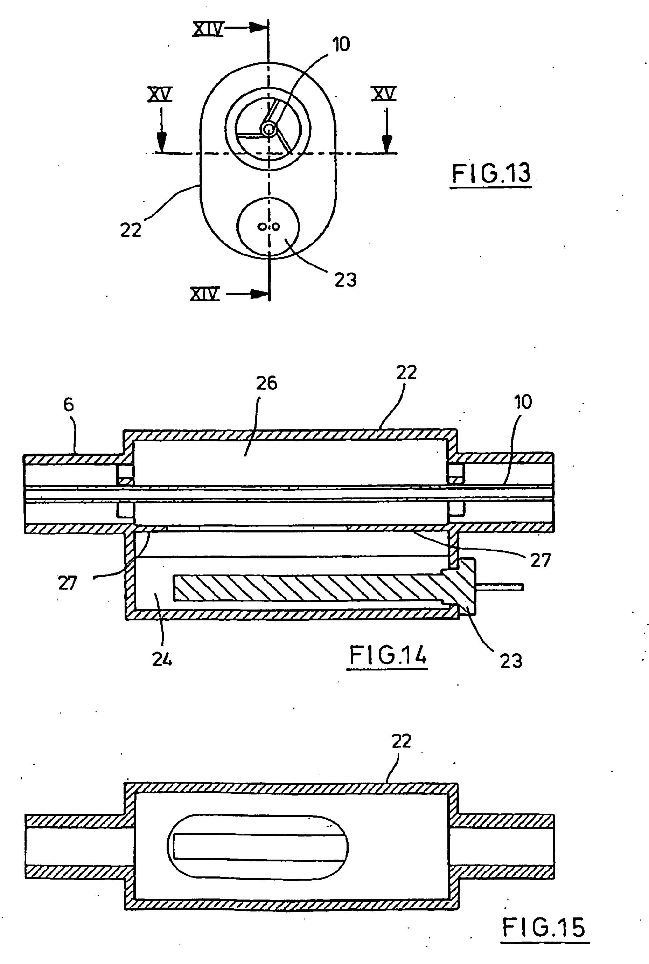

Fig. 13 zeigt eine andere Ausführungsform des Atemgasbefeuchters (22), dessen äußeres Erscheinungsbild im wesentlichen identisch zur Ausführungsform in Fig. 11 gestaltet ist. Die konstruktiven Unterschiede sind aber aus der Vertikalschnittdarstellung in Fig. 14 zu erkennen. Das Flüssigkeitsreservoir (24) ist hier durch Seitenstege (27) vom Durchgangsraum '(26) abgetrennt. Die Seitenstege (27) weisen den Vorteil auf, daß auch bei einer Schrägstellung des Gerätegehäuses (15) bis zu einem vorgebbaren Winkel ein Übertritt von Befeuchtungsflüssigkeit aus dem Flüssigkeitsreservoir (24) in den Durchgangsraum (26) und von diesem in den weiteren Bereich des Gerätegehäuses (15) hinein vermieden ist. Es wird somit eine erhöhte Betriebssicherheit bereitgestellt.

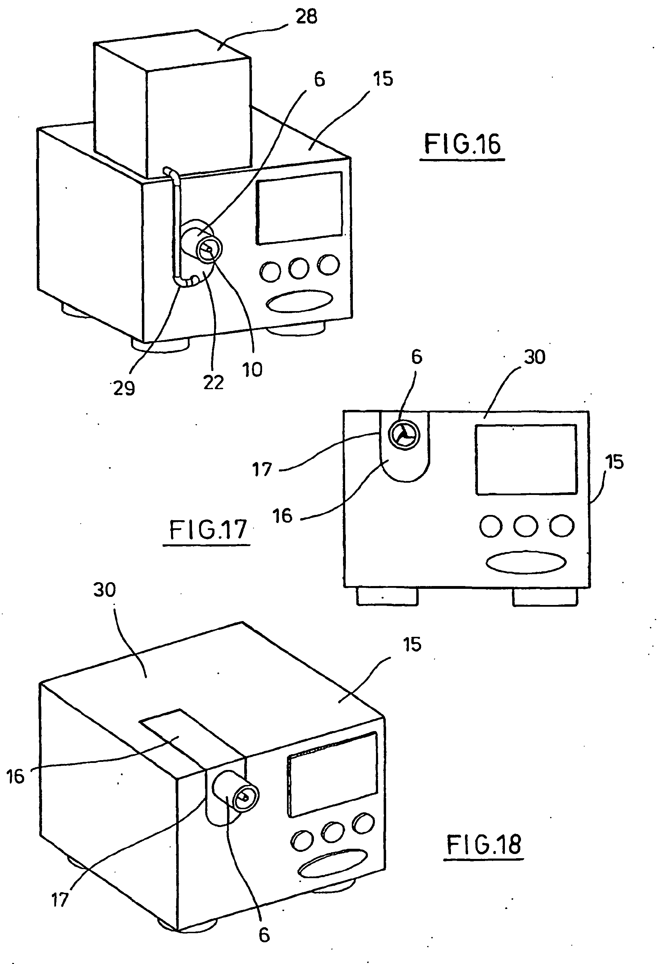

Fig. 16 zeigt ein Gerätegehäuse (15) mit eingesetztem Atemgasbefeuchter (22) sowie einem Vorratsbehälter (28) für Befeuchtungsflüssigkeit, der außenseitig am Gerätegehäuse (15) angeordnet ist. Beim dargestellten Ausführungsbeispiel ist der Vorratsbehälter (28) oben auf dem Gerätegehäuse (15) positioniert und über eine Außenleitung (29) mit dem Flüssigkeitsreservoir (24) des Atemgasbefeuchters (22) verbunden.

Fig. 17 zeigt eine gegenüber der Ausführungsform in Fig. 2 abgewandelte Konstruktion. Der Modulschacht (17) ist hierbei derart positioniert, daß der Modulschacht (17) bis in den Bereich einer Oberseite (30) des Gerätegehäuses (15) reicht. Eine Oberseite des verbindungsmoduls (16) verläuft hiermit etwa auf einem Höhenniveau wie die Oberseite (30). Alternativ wäre es ebenfalls denkbar, eine Anordnung des Modulschachtes (17) derart vorzunehmen, daß eine entsprechende Begrenzungsfläche des Verbindungsmoduls (16) im Bereich einer Seitenfläche des Gerätegehäuses (15) verläuft.

Fig. 18 veranschaulicht in einer perspektivischen Darstellung die Anordnung des Modulschachtes (17) sowie des Verbindungsmoduls (16) gemäß Fig. 17. Die im wesentlichen plane Anordnung der Oberseite (30) des Gerätegehäuses (15) sowie der entsprechenden Oberseite des Verbindungsmoduls (16) sind hier nochmals zu erkennen.

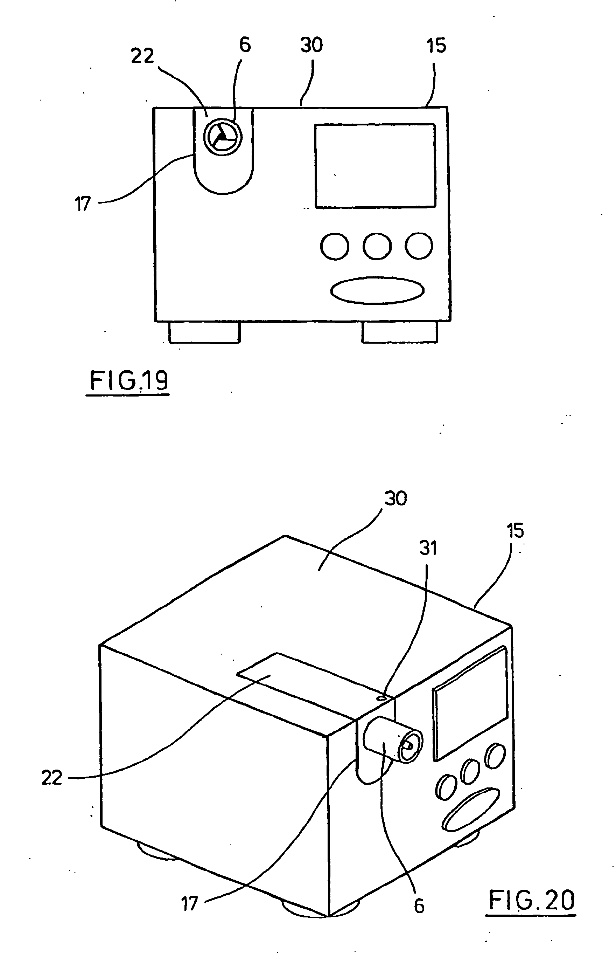

Fig. 19 und Fig. 20 entsprechen im wesentlichen den Darstellungen in Fig. 17 und Fig. 18, es wurde allerdings jeweils das verbindungsmodul (16) durch den Atemgasbefeuchter (22) ausgetauscht. Fig. 20 zeigt zusätzlich im Bereich der Oberseite des Atemgasbefeuchters (22) einen versorgungsanschluß (31), der zur Verbindung mit dem Vorratsbehälter (28) vorgesehen ist.

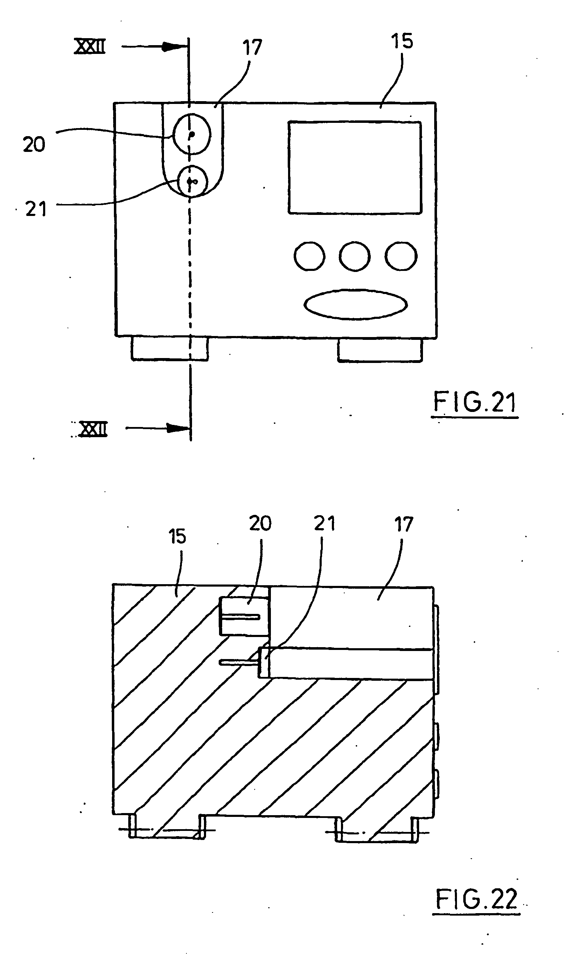

Fig. 21 zeigt das Gerätegehäuse (15) gemäß den Figuren 17 bis 20 nach einem Herausnehmen des verbindungsmoduls (16) bzw. des Atemgasbefeuchters (22). Innerhalb des Modulschachtes (17) sind wieder die Luftkupplung (20) sowie die Elektrokupplung (21) zu erkennen.

Fig. 22 veranschaulicht, daß die Gestaltung des Modulschachtes (17) auch bei dieser Ausführungsform im wesentlichen gleich zur bereits erläuterten Ausführungsform konstruiert ist. Unterschiedlich ist im wesentlichen die Gestaltung der oberen Begrenzung, die hier flach und nicht gekrümmt verläuft.

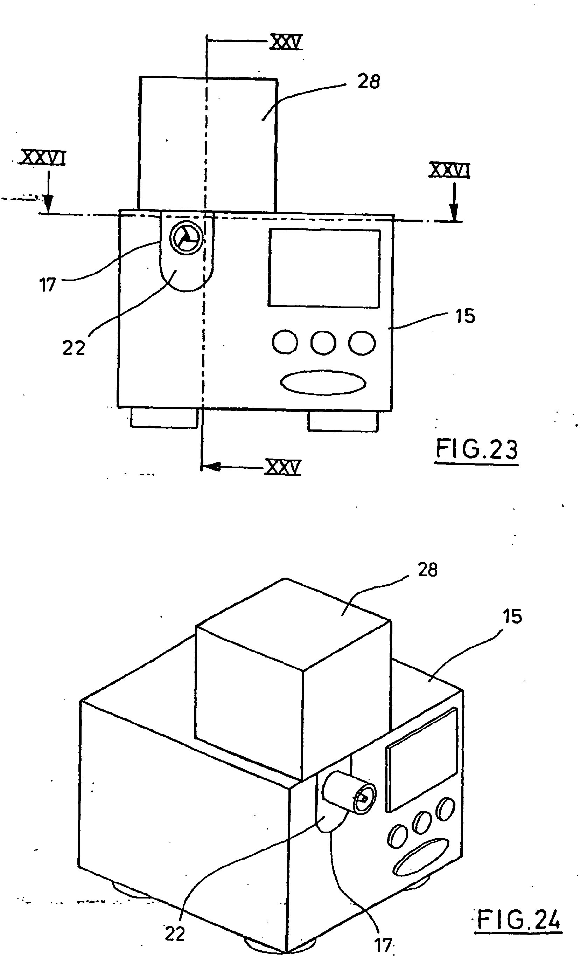

Fig. 23 und Fig. 24 zeigen das Gerätegehäuse (15) gemäß Fig. 19 und Fig. 20 nach einem Aufsetzen des vorratsbehälters (28). Es sind bei dieser Ausführungsform keine externen verbindungsleitungen erforderlich, da der Vorratsbehälter (28) mit einem geeigneten Kupplungsstück direkt in den versorgungsanschluß (31) eingreift.

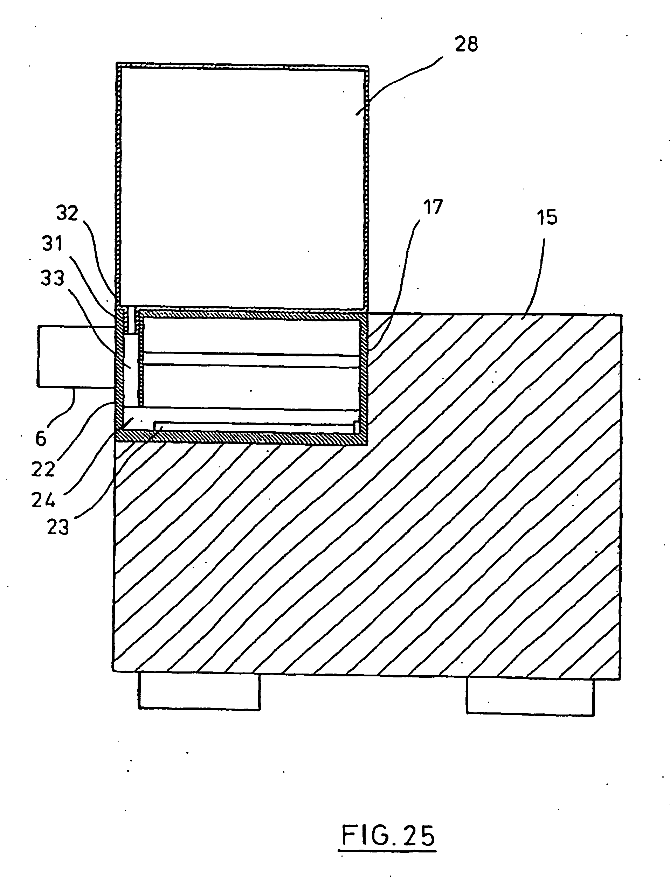

Aus Fig. 25 ist die Verbindung des Vorratsbehälters (28) mit dem Atemgasbefeuchter (22) zu erkennen. Der vorratsbehälter (28) greift hierbei mit einem Stutzen (32) in den versorgungsanschluß (31) ein. Durch den Atemgasbefeuchter (22) hindurch erstreckt sich vom Versorgungsanschluß (31) bis zum Flüssigkeitsreservoir (24) eine Verbindungsleitung (33).

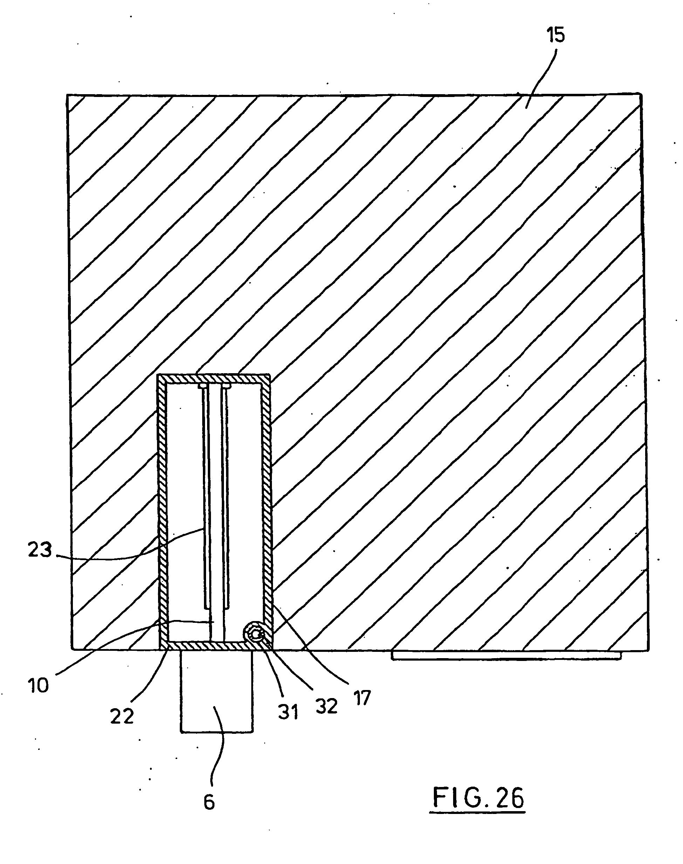

Fig. 26 veranschaulicht in einer Horizontalschnittdarstellung nochmals die Positionierung des Heizelementes (23) innerhalb des Atemgasbefeuchters (22) sowie das Ineinandergreifen des Stutzens (32) sowie des versorgungsanschlusses. (31).

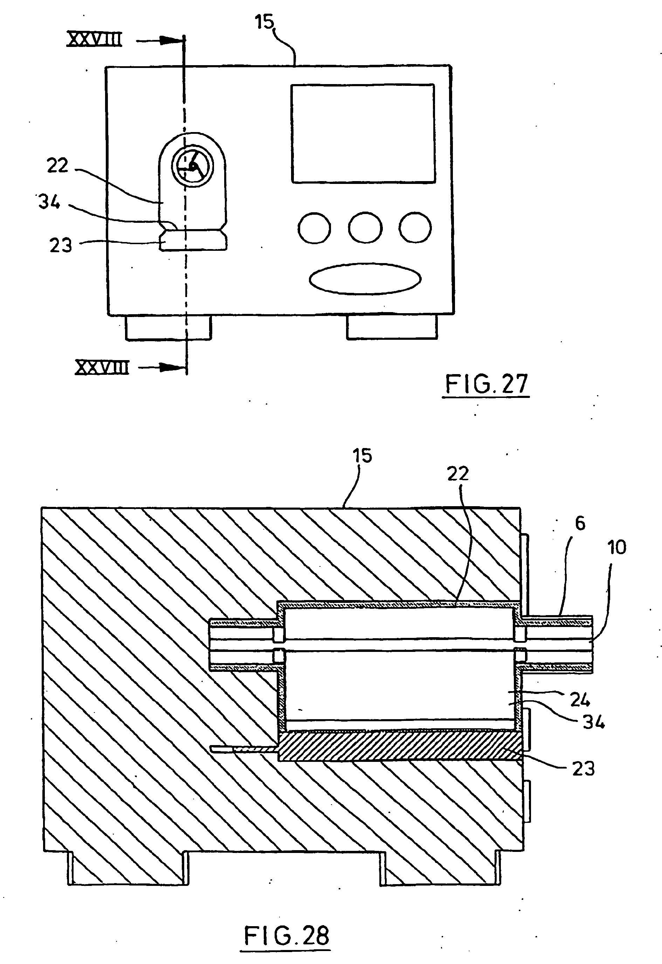

Fig. 27 zeigt ein Beispiel, bei der innerhalb des Modulschachtes (17) das Heizelement (23) getrennt vom Atemgasbefeuchter (22) angeordnet ist. Das Heizelement (23) weist hier eine plattenartige Gestaltung auf und das Flüssigkeitsreservoir (24) ist im Bereich seiner Unterseite von einer wärmeleitenden Platte (34) begrenzt, die beispielsweise aus Metall ausgebildet ist. Die Konstruktion gemäß Fig. 27 ist in dem Vertikalschnitt gemäß Fig. 28 nochmals veranschaulicht. Auch bei diesem Beispiel ist es denkbar, beispielsweise nicht dargestellte Seitenstege (27) zu verwenden, um ein Austreten von Flüssigkeit aus dem Flüssigkeitsreservoir (24) zu verhindern.

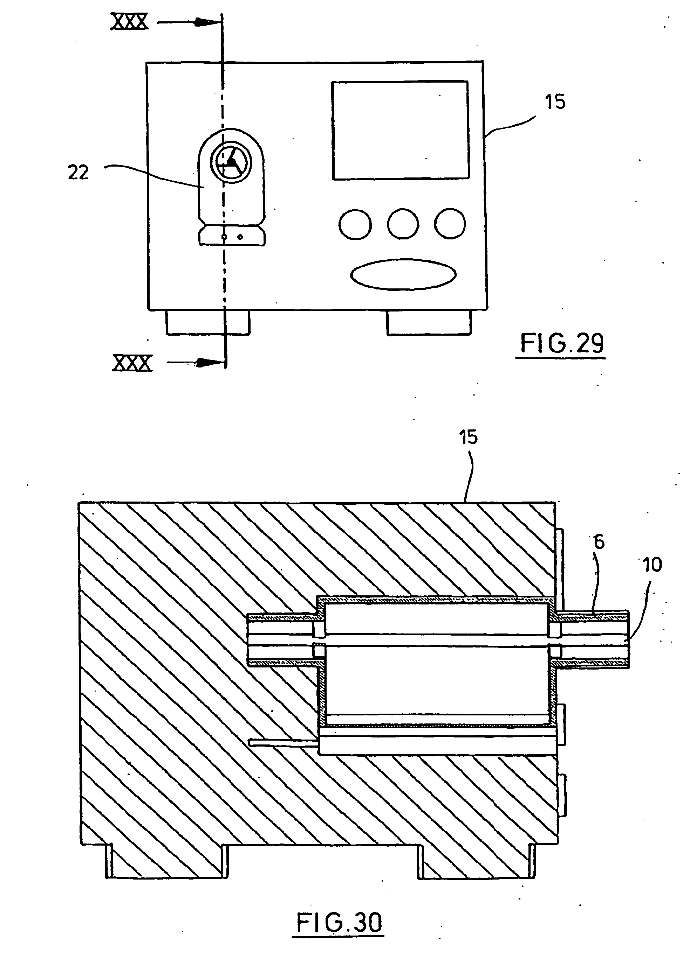

Fig. 29 und Fig. 30 zeigen die Konstruktion gemäß Fig. 27 und Fig. 28 bei herausgenommenem Heizelement (23). In diesem Beispiel kann ein Betrieb ohne Atemgasbefeuchtung erfolgen. Der Atemgasbefeuchter (22) übt hiermit ohne eingeschobenes Heizelement (23) gleichzeitig die Funktion des verbindungsmoduls (16) aus.

Alternativ zu den als Auslaufsicherung beschriebenen Seitenstegen (27) ist es grundsätzlich auch denkbar, das Flüssigkeitsreservoir (24) durch eine hydrophobe Membran vom Durchgangsraum (26) abzutrennen. Ebenfalls ist der Einsatz von Ventilen möglich. Alternativ zu den in den zeichnungen dargestellten Beispielen, bei denen der Atemgasbefeuchter (22) vollständig innerhalb des Gerätegehäuses (15) versenkt ist, ist es auch möglich, daß der Atemgasbefeuchter (22) teilweise aus dem Gerätegehäuse (15) herausragt.

Alternativ zu dem in den Zeichnungen dargestellten von außen zugänglichen Modulschacht (17) ist es auch denkbar, ein leicht zu demontierendes Gerätegehäuse (15) zu verwenden und einen Austausch der erforderlichen Baugruppen innerhalb des Gerätes vorzunehmen. Ein derartiger Austausch kann durch die Verwendung eines Montagerahmens unterstützt werden, auf dem das Gehäuse aufsitzt. Die Verwendung eines Montagerahmens erleichtert in einfacher Weise den Austausch von Modulen oder eine Ergänzung der Gerätefunktion durch weitere Module.

Ein weiteres Beispiel besteht darin, zwei unterschiedliche Gerätegehäuse (15) vorzusehen, wobei das eine Gerätegehäuse (15) für einen Betrieb ohne Vorratsbehälter (28) und ohne Atemgasbefeuchter (22) vorgesehen ist, und das andere Gerätegehäuse (15) derart groß dimensioniert ist, daß innerhalb des Gerätegehäuses (15) sowohl der Atemgasbefeuchter (22) als auch der Vorratsbehälter (28) anordbar sind.

Nachfolgend werden diverse Ausführungsbeispiele beschrieben, die alternativ oder ergänzend bezüglich der bereits erläuterten Beispiele zum Einsatz kommen können. Die Öffnung des Modulschachtes kann beispielsweise im wesentlichen in einer Fläche an der Oberseite der Gehäuseeinrichtung angeordnet sein. Es ist aber auch möglich, daß sich die Öffnung des Modulschachtes im wesentlichen in der Bodenfläche der Gehäuseeinrichtung oder in der Seitenfläche der Gehäuseeinrichtung befindet. Ebenfalls ist es denkbar, daß sich die Öffnung des Modulschachtes im wesentlichen in der Frontfläche der Gehäuseeinrichtung befindet oder im wesentlichen in einer der Seitenflächen und in einer Fläche an der Oberseite der Gehäuseeinrichtung angeordnet ist. Schließlich ist auch daran gedacht, daß sich die Öffnung des Modulschachtes im wesentlichen in der Frontfläche und in einer Fläche an der Oberseite der Gehäuseeinrichtung, im wesentlichen in zwei Seitenflächen der Gehäuseeinrichtung oder im wesentlichen in einer der Seitenflächen und in der Bodenfläche der Gehäuseeinrichtung befindet. Schließlich ist es auch möglich, daß sich die Öffnung des Modulschachtes im wesentlichen in der Frontfläche und in der Bodenfläche der Gehäuseeinrichtung oder im wesentlichen in einem Eckbereich der Gehäuseeinrichtung befindet.

Die Fügerichtung des Moduls kann sich im wesentlichen senkrecht zu einer der die Modulöffnung enthaltenden Flächen erstrecken. Es ist ebenfalls möglich, daß das Fügen des Moduls im wesentlichen in einer Schwenkbewegung erfolgt.

Das Modul kann in seiner eingesetzten Stellung lösbar verriegelt sein. Ebenfalls ist daran gedacht, daß das Modul durch einen Kraft- oder Reibschluß in seiner gefügten Position gehalten wird. Darüber hinaus ist daran gedacht, daß an der Gehäuseeinrichtung und/oder an dem Modul Elemente angebracht sind, die das Ausrichten und zentrieren des Moduls zum Modulschacht beim Fügen des Moduls erleichtern. An der Gehäuseeinrichtung bzw. am Modulschacht und/oder am Modul können Elemente angebracht sein, die der Führung des Moduls beim Fügen dienen. Die Führungselemente können als Nut und Feder ausgebildet sein.

Das Modul und der Modulschacht können so ausgebildet sein, daß eine eindeutige Orientierung des Moduls zum Modulschacht für das Einsetzen des Moduls erforderlich ist. Darüber hinaus können das Modul und der Modulschacht so ausgebildet sein, daß das Einsetzen des Moduls unter mindestens zwei verschiedenen Orientierungen des Moduls zum Modulschacht möglich ist.

Durch das Modul kann sich ein röhrenförmiges Element zum Schlauchanschluß hin erstrecken, das dem Anschluß einer Druckmeßleitung dient. In dem Modulschacht können wahlweise eines mehrerer verschiedener Module eingesetzt werden.

Im Bereich eines zur Atemluftbefeuchtung vorgesehenen Moduls befindet sich ein Bereich zur Aufnahme einer Flüssigkeit, um die durch das Modul strömende Atemluft anzufeuchten. Ein Auslaufen von Flüssigkeit aus dem Modul heraus bis zu einem vorgebbaren Neigungswinkel kann von einer Auslaufsicherung verhindert werden. Als Auslaufsicherung kann eine hydrophobe Membran oberhalb der Flüssigkeit verwendet werden. Die hydrophobe Membran kann im Bereich des Schlauchanschlußstutzens und/oder im Bereich der fördereinrichtungsseitigen Luftkupplung angeordnet werden.

Alternativ ist es auch möglich, daß als Auslaufsicherung ein Ventil im Bereich des Schlauchanschlußstutzens oder im Bereich der fördereinrichtungsseitigen Luftkupplung verwendet wird. Das Ventil kann als ein klappbares Element ausgebildet sein, daß durch den Luftstrom der Einrichtung zur Förderung des Atemgases geöffnet wird und sich schließt, wenn kein Luftstrom vorhanden ist. Das Ventil kann aus einem elastomeren Werkstoff, beispielsweise Silikon, ausgebildet sein.

Gemäß eines weiteren Beispiels ist daran gedacht, daß die Auslaufsicherung als eine Wand ausgebildet ist, die sich im Modul mindestens bereichsweise oberhalb der Flüssigkeit erstreckt. Die Auslaufsicherung kann auch als ein tubusförmiges Element ausgebildet sein, das sich in das Modul hineinerstreckt.

zur Beheizung der im Befeuchtungsmodul befindlichen Flüssigkeit wird eine spezielle Heizeinrichtung verwendet. Die Beheizung kann durch ein in die Flüssigkeit hineinragendes Element erfolgen. Das Element kann als ein elektrisches Heizelement ausgebildet sein. Im Modulschacht können elektrische Kontaktelemente angeordnet werden, die beim Einsetzen des Moduls in den Modulschacht einen elektrischen Kontakt zu den Anschlußorganen des elektrischen Heizelementes herstellen.

Das elektrische Heizelement kann auch außerhalb des Moduls angeordnet sein und die Beheizung der Flüssigkeit erfolgt über das in die Flüssigkeit hineinragende Element durch Wärmeleitung. Ebenfalls ist daran gedacht, daß das elektrische Heizelement als ein gesondertes Modul ausgebildet ist, das bei Bedarf in einer Aufnahme im Bereich des Modulschachtes eingesetzt werden kann. Ebenfalls ist es möglich, daß die Beheizung der Flüssigkeit durch ein unterhalb des Moduls angeordnetes Heizelement erfolgt. Das Modul weist hierzu einen thermisch leitfähigen Boden auf, durch den die Wärmeübertragung vom Heizelement an die Flüssigkeit erfolgt.

Gemäß eines weiteren Ausführungsbeispiels kann die Beheizung der Flüssigkeit durch Wärmestrahlung erfolgen. Ebenfalls kann die.Beheizung durch eine Induktionsheizung realisiert werden. Bei sämtlichen vorgeschlagenen Heizelementen können diese im Bereich gesonderten Module angeordnet werden, die bei Bedarf in eine Aufnahme des Modulschachtes eingesetzt werden.

Bei Verwendung eines außerhalb der Gehäuseeinrichtung angeordneten Vorratsbehälters ist es möglich, diesen mindestens bereichsweise in lotrechter Richtung oberhalb des Gerätes anzuordnen. Ebenfalls ist daran gedacht, den Vorratsbehälter mindestens bereichsweise neben dem Gerätegehäuse oder mindesten bereichsweise in lotrechter Richtung unterhalb des Gerätegehäuses zu positionieren.

Der Vorratsbehälter kann am Gerätegehäuse fixiert werden. Die Fixierung kann beispielsweise durch eine verriegelung realisiert werden. Die Fixierung des Vorratsbehälters kann auch durch das Einsetzen oder das Aufsetzen von Fixierelementen und/oder Zentrierelementen des vorratsbehälters in oder auf komplementär ausgebildete Elemente des Gerätegehäuses oder des Moduls realisiert werden.

Die Vorrichtung kann eine Einrichtung zum Fördern der Flüssigkeit aus dem vorratsbehälter in den Befeuchtungbereich des Moduls umfassen. Die Förderung der Flüssigkeit kann durch eine sich zwischen dem Vorratsbehälter und dem Modul erstreckende Fluidleitung hindurch erfolgen. Die Fluidleitung kann hierbei den Flüssikeitsvorratsbehälter und das Modul außerhalb des Gerätegehäuses verbinden. Die Fluidverbindung kann durch das Aufsetzen oder das Einsetzen des vorratsbehälters auf oder in einen versorgungsanschluß des Moduls hergestellt werden.

Im Bereich der Fluidleitung und/oder im Bereich des Fluidleitungsanschlusses des Moduls kann mindestens ein Ventil angeordnet sein, das beim Zustandekommen der Verbindung zwischen dem vorratsbehälter und dem Modul bzw. zwischen dem Flüssigkeitsvorratsbehälter und der Fluidleitung bzw. zwischen der Fluidleitung und dem Modul einen Ventildurchgang öffnet und beim Trennen der verbindung den Durchgang wieder schließt, so daß ein unbeabsichtigtes Auslaufen von Flüssigkeit aus dem Vorratsbehälter heraus zumindest weitgehend verhindert wird.

Das Fördern der Flüssigkeit kann unter Ausnutzung der Schwerkraft erfolgen. Die Fluidleitung kann unterhalb des vorgesehenen Flüssigkeitsniveaus im Modul enden und der Transport der Flüssigkeit aus dem vorratsbehälter in den Befeuchtungsbereicht kann durch Belüftung des Vorratsbehälters erfolgen. Ebenfalls ist es möglich, im Bereich der Fluidleitung ein elektrisch gesteuertes Ventil anzuordnen, das die Fluidleitung öffnen und schließen kann.

Zur Erfassung eines Flüssigkeitsstandes im Modul kann ein Füllstandssensor verwendet werden. Das Ventil kann in Abhängigkeit vom innerhalb des Moduls erfaßten Flüssigkeitsstand seinen Durchgang öffnen und schließen. Gemäß einer weiteren Ausführungsform kann die Förderung der Flüssigkeit auch durch eine Pumpe erfolgen. Auch bei Verwendung einer Pumpe können die bereits erläuterten varianten hinsichtlich der verwendung von elektrisch steuerbaren Ventilen sowie der Füllstandserfassung eingesetzt werden.

Die Gehäuseeinrichtung wird konstruktiv derart ausgeführt, daß eine einfache Demontage unterstützt wird. Insbesondere ist daran gedacht, die Gehäuseeinrichtung derart zu konstruieren, daß eine manuelle Demontage ohne verwendung von Werkzeugen möglich ist. Eine Konstruktionsvariante zur Erreichung dieser Eigenschaften besteht darin, daß die Gehäuseteile durch verhaken miteinander verbunden werden. Die Verbindung der Gehäuseteile zueinander kann zumindest bereichsweise durch Druck oder Zug im Bereich eines oder mehrerer der Haken gelöst werden.

Gemäß weiterer Ausführungsbeispiele ist daran gedacht, daß die Gehäuseteile über eine Durchführung von Schwenkbewegungen montiert sowie demontiert werden. Darüber hinaus ist es möglich, daß die Gehäuseteile mit einer oder mehreren annähernd linearen Bewegungen montiert bzw. demontiert werden.

Die demontierbaren Gehäuseteile können an anderen Gehäuseteilen befestigt werden. Innerhalb des Gehäuses kann ein Montagerahmen angeordnet werden. Der Montagerahmen kann beispielsweise aus einem relativ harten Kunststoff, einem verstärkten Kunststoff, einem faserverstärkten Kunststoff oder aus Metall bestehen. Die demontierbaren Gehäuseteile können auch am Montagerahmen festgelegt werden. Im Inneren des Gehäuses können die Einrichtung zur Förderung des Atemgases sowie andere Komponenten, beispielsweise Elektronik-Platinen, Netzteil, Schalldämmbox, Luftkanäle, Filtereinrichtung, Anfeuchter oder Heizelement am Montagerahmen befestigt werden. Im Inneren des Gehäuses können auch ein oder mehrere Komponenten als separate Module aufgebaut sein.

Eine Montage und eine Demontage der Komponenten innerhalb des Gehäuses kann einfach durchgeführt werden. Insbesondere ist daran gedacht, daß ein Austausch der Module manuell ohne Verwendung von Werkzeugen möglich ist. Alternativ zu einer Montage und Demontage einzelner Module ist es auch möglich, das System mit an die jeweiligen Funktionseigenschaften angepaßten Modulen zu versehen, die die jeweils erforderlichen Fähigkeiten aufweisen. In Abhängigkeit von den konkreten Anwendungsanforderungen können ein oder mehrere Module eingesetzt werden. Darüber hinaus ist auch daran gedacht, daß eine nachträgliche Montage von Modulen unterstützt wird.

## Patentansprüche

1. Verfahren zur zufuhr von Atemgas, bei dem das Atemgas von einer Gasfördereinrichtung mit einem Förderdruck versehen wird sowie bei dem das Atemgas innerhalb eines die Gasfördereinrichtung aufnehmenden Gerätegehäuses (15) entlang eines Luftkanals gefördert wird, sowie bei dem im Bereich des Luftkanals in einem Modulschacht (17) ein Modul (22) zur Durchleitung des Atemgases positioniert ist, wobei das Modul (22) für den Modulschacht (17) mit einer in Richtung der Gasfördereinrichtung angeordneten Luftkupplung (20), sowie mit einer in Richtung auf einen Atemschlauch angeordneten Anschlusskupplung versehen ist und im Bereich des Moduls (22) eine Befeuchtung des Atemgases durchgeführt wird, **dadurch gekennzeichnet, dass** das Modul als Atemgasbefeuchtermodul (22) ausgestattet ist und herausnehmbar in einem Modulschacht (17) des Gerätegehäuses (15) angeordnet ist, dass ein Heizelement (23) innerhalb des Atemgasbefeuchtermoduls (22) angeordnet ist und dass das Heizelement (23) in eine innerhalb des Atemgasbefeuchtermoduls (22) befindliche Flüssigkeit hineinragt.

2. Verfahren nach Anspruch 1, **dadurch gekennzeichnet, dass** mindestens ein Teil einer zur Befeuchtung des Atemgases verwendeten Flüssigkeit in einem Vorratsbehälter (28) außerhalb des Gerätegehäuses (15) bereitgestellt wird und dass mindestens ein Teil der Flüssigkeit aus dem Vorratsbehälter (28) zu einem innerhalb des Gerätegehäuses (15) positionierten Atemgasbefeuchtermoduls (22) geleitet wird.

3. Verfahren nach einem der Ansprüche 1 bis 2, **dadurch gekennzeichnet, dass** die Flüssigkeit mit einer Bewegungskomponente in lotrechter Richtung von oben in Richtung auf das Atemgasbefeuchtermodul (22) geleitet wird.

4. Verfahren nach einem der Ansprüche 1 bis 2, **dadurch gekennzeichnet, dass** die Flüssigkeit mit einer Bewegungskomponente auf einem im Wesentlichen horizontalen Höhenniveau zum Atemgasbefeuchtermodul (22) geleitet wird.

5. Verfahren nach einem der Ansprüche 1 bis 2, **dadurch gekennzeichnet, dass** die Flüssigkeit mit einer Bewegungskomponente in lotrechter Richtung von unten in Richtung auf das Atemgasbefeuchtermodul (22) geleitet wird.

6. Verfahren nach einem der Ansprüche 1 bis 5, **dadurch gekennzeichnet, dass** eine Verbindung des Vorratsbehalters (28) mit dem Atemgasbefeuchtermodul (22) ventilgesteuert erfolgt.

7. Verfahren nach einem der Ansprüche 1 bis 6, **dadurch gekennzeichnet, dass** das Atemgasbefeuchtermodul (22) elektrisch beheizt wird.

8. Verfahren nach einem der Ansprüche 1 bis 7, **dadurch gekennzeichnet, dass** eine Beheizung unmittelbar innerhalb der Flüssigkeit durchgeführt wird.

9. Verfahren nach einem der Ansprüche 1 bis 7, **dadurch gekennzeichnet, dass** Heizenergie der Flüssigkeit von einem wärmeüberträger zugeführt wird.

10. Verfahren nach einem der Ansprüche 1 bis 9, **dadurch gekennzeichnet, dass** ein Auslaufen von Flüssigkeit aus dem Atemgasbefeuchtermodul (22) heraus bis zu einem vorgebbaren Neigungswinkel von einer Auslaufsicherung verhindert wird.

11. Verfahren nach einem der Ansprüche 1 bis 10, **dadurch gekennzeichnet, dass** eine Energieversorgung des Heizelementes (23) in Abhängigkeit von einer Detektion eines angeschlossenen Vorratsbehälters (28) durchgeführt wird.

12. Verfahren nach einem der Ansprüche 1 bis 11, **dadurch gekennzeichnet, dass** die Flüssigkeitszufuhr zum Atemgasbefeuchtermodul (22) ventilgesteuert durchgeführt wird.

13. Verfahren nach einem der Ansprüche 1 bis 12, **dadurch gekennzeichnet, dass** im Bereich des Atemgasbefeuchtermoduls (22) eine Füllstandserfassung durchgeführt wird.

14. Verfahren nach einem der Ansprüche 1 bis 13, **dadurch gekennzeichnet, dass** dem Atemgasbefeuchtermodul (22) die Flüssigkeit von einer Pumpe gesteuert zugeführt wird.

15. Vorrichtung zur Beatmung (1), die eine Atemgasversorgung sowie einen Luftkanal aufweist, der innerhalb eines die Atemgasversorgung aufnehmenden Gerätegehäuses (15) angeordnet ist, sowie bei der mindestens ein Teil des Luftkanals als ein Modulschacht (17) ausgebildet ist, in den ein Modul (22) zur Durchleitung des Atemgases eingesetzt ist, wobei das Modul (22) für den Modulschacht (17) als ein Atemgasbefeuchter ausgebildet ist, das eine der Atemgasversorgung zugewandt angeordnete Luftkupplung (20) sowie eine einem Atemgasschlauch zugewandt angeordnete Auslasskupplung aufweist, **dadurch gekennzeichnet, dass** das Modul (22) als Atemgasbefeuchtermodul (22) ausgestattet ist und herausnehmbar in dem Modulschacht (17) des Gerätegehäuses (15) angeordnet ist, dass ein Heizelement (23) innerhalb des Atemgasbefeuchtermoduls (22) angeordnet ist und dass das Heizelement (23) in eine innerhalb des Atemgasbefeuchtermoduls (22) befindliche Flüssigkeit hineinragt.

16. Vorrichtung nach Anspruch 15, **dadurch gekennzeichnet, dass** ein Flüssigkeitsreservoir (24) des Atemgasbefeuchtermoduls (22) mit einem Vorratsbehälter (28) koppelbar ist, der außerhalb des Gerätegehäuses (15) angeordnet ist und dass der Vorratsbehälter (28) außenseitig am Gerätegehäuse (15) fixierbar angeordnet ist.

17. Vorrichtung nach einem der Ansprüche 15 bis 16, **dadurch gekennzeichnet, dass** der Vorratsbehälter (28) mindestens bereichsweise seitlich neben dem Gerätegehäuse (15) angeordnet ist.

18. Vorrichtung nach einem der Ansprüche 15 bis 17, **dadurch gekennzeichnet, dass** der Vorratsbehälter (28) über einen Versorgungsanschluß (31) unmittelbar an den Atemgasbefeuchtermodul (22) angekoppelt ist.

19. Vorrichtung nach Anspruch 18, **dadurch gekennzeichnet, dass** der Vorratsbehälter (28) mit einem Stutzen (32) in einen Versorgungsanschluß (31) des Atemgasbefeuchters (22) eingreift.

20. Vorrichtung nach einem der Ansprüche 15 bis 19, **dadurch gekennzeichnet, dass** der Vorratsbehälter (28) ventilgesteuert mit dem Flüssigkeitsreservoir (24) verbunden ist.

21. Vorrichtung nach einem der Ansprüche 15 bis 21, **dadurch gekennzeichnet, dass** innerhalb des Modulschachtes (17) ein Verbindungsmodul (16) für einen Betrieb ohne Atemgasbefeuchtung anordbar ist.

22. Vorrichtung nach einem der Ansprüche 15 bis 21, **dadurch gekennzeichnet, dass** eine Heizung des Luftbefeuchters (22) als ein elektrisches Heizelement (23) ausgebildet ist.

23. Vorrichtung nach einem der Ansprüche 15 bis 22, **dadurch gekennzeichnet, dass** das Heizelement (23) als ein Heizstab ausgebildet ist.

24. Vorrichtung nach einem der Ansprüche 15 bis 23, **dadurch gekennzeichnet, dass** das Heizelement (23) als ein vom Atemgasbefeuchtermodul (22) trennbares separates Bauelement ausgebildet ist.

## Claims

1. Method of supplying breathable gas, in which said breathable gas is provided with a feed pressure by a gas feed apparatus, and in which said breathable gas is fed along an air duct inside an appliance housing (15) which receives said gas feed apparatus, and in which a module (22) for conducting-through the breathable gas is positioned in a module shaft (17) in the region of the air duct, wherein the module (22) for the module shaft (17) is provided with an air coupling (20) which is arranged in the direction of the gas feed apparatus, and also with a connecting coupling which is arranged in the direction of a breathing hose, and moistening of the breathable gas is carried out in the region of the module (22), **characterised in that** the module is equipped as a breathable gas moisturiser module (22) and is arranged, in a removable manner, in a module shaft (17) belonging to the appliance housing (15), that a heating element (23) is arranged inside the breathable gas moisturiser module (22), and that the heating element (23) projects into a liquid located inside said breathable gas moisturiser module (22).

2. Method according to Claim 1, **characterised in that** at least some of a liquid which is used for moisturising the breathable gas is made available in a storage container (28) outside the appliance housing (15), and that at least some of said liquid is conducted from said storage container (28) to a breathable gas moisturiser module (22) positioned inside said appliance housing (15).

3. Method according to either of Claims 1 or 2, **characterised in that** the liquid is conducted in the direction of the breathable gas moisturiser module (22) from above, with a component of motion in the vertical direction.

4. Method according to either of Claims 1 or 2, **characterised in that** the liquid is conducted to the breathable gas moisturiser module (22) with a component of motion on a vertical level which is substantially horizontal.

5. Method according to either of Claims 1 or 2, **characterised in that** the liquid is conducted in the direction of the breathable gas moisturiser module (22) from below with a component of motion in the vertical direction.

6. Method according to one of Claims 1 to 5, **characterised in that** a connection of the storage container (28) to the breathable gas moisturiser module (22) takes place in a valve-controlled manner.

7. Method according to one of Claims 1 to 6, **characterised in that** the breathable gas moisturiser module (22) is heated electrically.

8. Method according to one of Claims 1 to 7, **characterised in that** heating is carried out directly within the liquid.

9. Method according to one of Claims 1 to 7, **characterised in that** heating energy is supplied to the liquid by a heat-exchanger.

10. Method according to one of Claims 1 to 9, **characterised in that** spillage of liquid from the breathable gas moisturiser module (22) is prevented, up to a pre-determinable angle of inclination, by a spill-proofing device.

11. Method according to one of Claims 1 to 10, **characterised in that** the supplying of energy to the heating element (23) is carried out in dependence upon detection of a connected storage container (28).

12. Method according to one of Claims 1 to 11, **characterised in that** the supplying of liquid to the breathable gas moisturiser module (22) is carried out in a valve-controlled manner.

13. Method according to one of Claims 1 to 12, **characterised in that** detection of the filling level is carried out in the region of the breathable gas moisturiser module (22).

14. Method according to one of Claims 1 to 13, **characterised in that** the liquid is supplied to the breathable gas moisturiser module (22) in a controlled manner by a pump.

15. Device (1) for artificial respiration, which has a breathable gas supply and also an air duct which is arranged inside an appliance housing (15) which receives said breathable gas supply, and in which device at least part of the air duct is constructed as a module shaft (17) in which a module (22) for conducting-through the breathable gas is inserted, wherein the module (22) for the module shaft (17) is constructed as a breathable gas moisturiser which has an air coupling (20) which is arranged in a manner facing towards the breathable gas supply, and also an outlet coupling which is arranged in a manner facing towards a breathable gas hose, **characterised in that** the module (22) is equipped as a breathable gas moisturiser module (22) and is arranged, in a removable manner, in the module shaft (17) of the appliance housing (15), that a heating element (23) is arranged inside the breathable gas moisturiser module (22), and that the heating element (23) projects into a liquid located inside said breathable gas moisturiser module (22).

16. Device according to Claim 15, **characterised in that** a liquid reservoir (24) belonging to the breathable gas moisturiser module (22) can be coupled to a storage container (28) which is arranged outside the appliance housing (15), and that said storage container (28) is arranged in a fixable manner on the outside of said appliance housing (15).

17. Device according to either of Claims 15 or 16, **characterised in that** the storage container (28) is arranged laterally beside the appliance housing (15), at least in certain regions.

18. Device according to one of Claims 15 to 17, **characterised in that** the storage container (28) is coupled directly to the breathable gas moisturiser module (22) via a supply connection (31).

19. Device according to Claim 18, **characterised in that** the storage container (28) engages, with the aid of a connecting piece (32), in a supply connection (31) belonging to the breathable gas moisturiser (22).

20. Device according to one of Claims 15 to 19, **characterised in that** the storage container (28) is connected to the liquid reservoir (24) in a valve-controlled manner.

21. Device according to one of Claims 15 to 20, **characterised in that** a connecting module (16) for operation without the moisturising of the breathing gas may be arranged inside the module shaft (21).

22. Device according to one of Claims 15 to 21, **characterised in that** a heating system belonging to the air-moisturiser (22) is constructed as an electric heating element (23).

23. Device according to one of Claims 15 to 22, **characterised in that** the heating element (23) is constructed as a heating bar.

24. Device according to one of Claims 15 to 23, **characterised in that** the heating element (23) is constructed as a separate structural element which can be separated from the breathable gas moisturiser module (22).

## Revendications

1. Procédé de distribution de gaz respirable, dans lequel le gaz respirable est pourvu, par un dispositif de transport de gaz, d'une pression de transport, et dans lequel le gaz respirable est transporté, le long d'un canal d'air, à l'intérieur d'un boîtier d'appareil (15), qui reçoit le dispositif de transport de gaz, ainsi que dans lequel un module (22) est positionné, pour le passage du gaz respirable, dans une cage de module (17), dans la région du canal d'air, sachant que le module (22), pour la cage de module (17), est pourvu d'un couplage d'air (20) ainsi que d'un couplage de raccordement, qui est disposé en direction d'un tuyau respiratoire, et que, dans la région du module (22) est effectuée une humidification du gaz respirable,
**caractérisé en ce que** le module est conçu en tant que module d'humidification de gaz respirable (22) et est agencé de manière amovible dans une cage de module (17) du boîtier d'appareil (15), qu'un élément de chauffage (23) est disposé à l'intérieur du module d'humidification de gaz respirable (22) et que l'élément de chauffage (23) fait saillie dans un liquide qui se trouve à l'intérieur du module d'humidification de gaz respirable (22).

2. Procédé selon la revendication 1,
**caractérisé en ce que**, dans un réservoir (28), à l'extérieur du boîtier d'appareil (15), au moins une partie d'un liquide, utilisé pour l'humidification du gaz respirable, est disponible et qu'au moins une partie du liquide est conduite du réservoir (28) à un module d'humidification de gaz respirable (22) qui est positionné à l'intérieur du boîtier d'appareil (15).

3. Procédé selon revendication 1 à 2,
**caractérisé en ce que** le liquide est conduit, d'en haut, avec une composante de déplacement dans la direction perpendiculaire, en direction du module d'humidification de gaz respirable (22).

4. Procédé selon l'une des revendications 1 à 2,
**caractérisé en ce que** le liquide est conduit au module d'humidification de gaz respirable (22), avec une composante de déplacement, à un niveau de hauteur horizontal.

5. Procédé selon l'une des revendications 1 à 2,
**caractérisé en ce que** le liquide est conduit d'en bas, avec une composante de déplacement, dans la direction perpendiculaire, en direction du module d'humidification de gaz respirable (22).

6. Procédé selon l'une des revendications 1 à 5,
**caractérisé en ce que** l'on procède à un raccordement du réservoir (28) au module d'humidification de gaz respirable (22).

7. Procédé selon l'une des revendications 1 à 6,
**caractérisé en ce que** le module d'humidification de gaz respirable (22) est chauffé électriquement.

8. Procédé selon l'une des revendications 1 à 7,
**caractérisé en ce que** le chauffage est effectué directement dans le liquide.

9. Procédé selon l'une des revendications 1 à 7,
**caractérisé en ce que** l'énergie de chauffage du liquide est amenée par un transmetteur de chaleur.

10. Procédé selon l'une des revendications 1 à 9,
**caractérisé en ce que** le débordement du liquide hors du module d'humidification de gaz respirable (22) est empêché par un dispositif de sécurité anti-débordement, jusqu'à un angle d'inclinaison qui peut être prédéterminé.

11. Procédé selon l'une des revendications 1 à 10,
**caractérisé en ce que** l'alimentation en énergie de l'élément de chauffage (23) est effectuée en fonction de la détection d'un réservoir (28) raccordé.

12. Procédé selon l'une des revendications 1 à 11,
**caractérisé en ce que** l'alimentation en liquide du module d'humidification de gaz respirable (22) est commandée par une soupape.

13. Procédé selon l'une des revendications 1 à 12,
**caractérisé en ce que**, dans la région du module d'humidification de gaz respirable (22), est effectuée une détection du niveau de remplissage.

14. Procédé selon l'une des revendications 1 à 13,
**caractérisé en ce que** le liquide est amené par commande d'une pompe au module d'humidification de gaz respirable (22).

15. Dispositif de ventilation respiratoire (1) qui est doté d'un système d'alimentation en gaz respirable ainsi que d'un canal d'air qui est disposé à l'intérieur d'un boîtier d'appareil (15) recevant ledit système d'alimentation en gaz respirable, ainsi que dans lequel au moins une partie du canal d'air est conçu en tant que cage de module (17), dans laquelle un module est inséré pour le passage du gaz respirable, sachant que le module (22) prévu pour la cage de module (17) est conçu en tant que dispositif d'humification du gaz respirable qui est doté d'un couplage d'air (20) orienté vers le système d'alimentation en gaz respirable ainsi que d'un couplage de sortie orienté vers un tuyau respiratoire,
**caractérisé en ce que** le module (22) est conçu en tant que module d'humidification de gaz respirable (22) et est agencé de manière amovible dans la cage de module (17) du boîtier d'appareil (15), qu'un élément de chauffage (23) est disposé à l'intérieur du module d'humidification de gaz respirable (22) et que ledit élément de chauffage (23) fait saillie dans un liquide qui se trouve à l'intérieur du module d'humidification de gaz respirable (22).

16. Dispositif selon la revendication 15,
**caractérisé en ce qu**' un réservoir de liquide (24) du module d'humidification de gaz respirable (22) peut être couplé avec un réservoir (28) qui est agencé à l'extérieur du boîtier d'appareil (15) et q u e ledit réservoir (28), disposé à l'extérieur, peut être fixé sur le boîtier d'appareil (15).

17. Dispositif selon l'une des revendications 15 à 16,
**caractérisé en ce que** le réservoir (28) est disposé latéralement, à côté du 15, au moins par sections.

18. Dispositif selon l'une des revendications 15 à 17,
**caractérisé en ce que** le réservoir (28) est directement couplé avec le module d'humidification de gaz respirable (22) au moyen d'un raccord d'alimentation (31).

19. Dispositif selon la revendication 18,
**caractérisé en ce que** le réservoir (28) s'engage, avec une tubulure (32) dans un raccord d'alimentation (31) du module d'humidification de gaz respirable (22).

20. Dispositif selon l'une des revendications 15 à 19,
**caractérisé en ce que** le réservoir (28) est relié, commandé par soupape, au réservoir de liquide (24).

21. Dispositif selon l'une des revendications 15 à 20,
**caractérisé en ce que**, pour le fonctionnement sans humidification du gaz respirable, un module de liaison (16) peut être installé à l'intérieur de la cage de module (17).

22. Dispositif selon l'une des revendications 15 à 21,
**caractérisé en ce qu**' un dispositif de chauffage du module d'humidification de gaz respirable (22) présente la forme d'un élément de chauffage électrique (23).

23. Dispositif selon l'une des revendications 15 à 22,
**caractérisé en ce que** l'élément de chauffage (23) présente la forme d'une barre chauffante.

24. Procédé selon l'une des revendications 15 à 23,
**caractérisé en ce que** l'élément de chauffage (23) présente la forme d'un composant qui peut être séparé du module d'humidification de gaz respirable (22).
